# EUROPEAN PATENT SPECIFICATION

(11) **EP 0 429 556 B1**
(45) Date of publication and mention of the grant of the patent: **11.09.1996**
(21) Application number: 90902589.2
(22) Date of filing: 25.01.1990
(51) Int. Cl.: C08J 5/06, C08J 5/08, A61K 6/083, A61K 6/087, A61K 6/09

(54) **REINFORCED COMPOSITE RESIN**
VERSTÄRKTES KOMPOSITHARZ
RESINE COMPOSITE RENFORCEE

(30) Priority: 27.01.1989 AU 2437/89
(43) Date of publication of application: 05.06.1991
(73) Proprietor: Hall, Neil Rex, Wahroonga New South Wales 2076 (AU); DOUBE, Christopher Philip, Lindfield, NSW 2070 (AU)
(72) Inventor: Hall, Neil Rex, Wahroonga New South Wales 2076 (AU); DOUBE, Christopher Philip, Lindfield, NSW 2070 (AU)
(74) Representative: Warren, Anthony Robert
(86) International application number: AU9000023
(87) International publication number: WO9008799

(56) References cited:
- EP-A- 0 193 003
- EP-A- 0 345 581
- AU-A- 5 843 786
- AU-B- 1 749 283
- AU-B- 6 921 681
- US-A- 4 244 993
- US-A- 4 770 935
- US-A- 4 778 722
- PATENTS ABSTRACTS OF JAPAN, C 544, page 83, JP,A, 63-162726 (Ube Ind Ltd) 6 July 1988 (06.07.88)
- DERWENT ABSTRACT Accession No. 88-180905/26, Class F01, JP,A, 63-120169 (Ube Industries KK) 24 May 1988 (24.05.88)

## Description

The invention relates to an improved, castable, synthetic resin material, suitable for use in dental and medical restorations, as a replacement for dental enamel and hydroxyapatite bone material.

Castable, synthetic resin materials currently on the market are perceived to be brittle in performance, particularly when they are compared with metals that have been traditionally used in dental and medical restorative applications. However, metal systems such as alloys and amalgams suffer from limited biocompatability and high heat and electrical conductivity, these deficiencies having negative health implications.

Dental composite resins have been developed from castable, synthetic resins especially for dental applications. Dental composite resins are characterized by extensive variation in the resin matrices utilized, ranging from acrylic through epoxy to recently developed urethane modified resins. Dental composite resin materials are easy to use and polish, they are photocurable and/or chemically curable and are relatively tough, strong and durable.

Dental composite resin materials are different from most castable, synthetic resin materials in that they are highly loaded (up to 90% and possibly further) with fine filler particles, and are predominantly photocured.

In general, dental composite resin materials include synthetic resins such as Bisphenol-A-diglycidyl dimethacrylate bis/GMA, urethane dimethacrylates, difunctional monomers such as Triethyleneglycol dimethacrylate TEGDMA; photoinitiators; amine accelerators; and polymerisation inhibitors and reinforcing fillers such as precipitated and pyrogenic silica, fine particles of glass (Ba, B, Sr, Al, Li, -SiO₂) silica (quartz) or ceramic. Dental composite resins are classified as conventional, microfine or hybrid depending on the reinforcing fillers used.

A typical hybrid formula is 15% bis/GMA, 15% TEGDMA, 50% pyrogenic silica, 20% Ba glass and <1% photoinitiators.

Biocompatibility is still being investigated, however, dental composite resins appear to be more biocompatible than metals.

The filler particles used are generally small (<5µm) so as to enable greater filler loadings and enhance physical properties such as polymerisation shrinkage, plain strain fracture toughness (K_{lC}) surface polishability and retained smoothness. Conventional dental composite resins with larger particles (<30µm) cause unacceptable surface roughness of restorations (fillings) when the inevitable surface wear of the matrix resin occurs.

Existing dental composite resins do not have the necessary physical properties to be universally endorsed as amalgam replacements.

The current invention relates to the finding that the strength and toughness of castable, synthetic resinMs can be further improved by the incorporation of fibres

Particles can be defined as having similar extent in three dimensions whereas fibres have substantially greater extent in one dimension compared to two dimensions, and platelets have substantially greater extent in two dimensions compared to one dimension.

In particular, the strength and toughness of dental composite resins and their acceptability as amalgam replacements can be improved without substantially reducing the acceptability of the dental composite resin for curing and surface finishing, by the incorporation of fibres, platelets or combinations thereof. Fibres and platelets produce greater K_{lC} than particles because of the higher energy required for debonding fibres or platelets compared to particles from a resin matrix. Hence more energy is required for fracture to occur.

Fibre reinforcement of castable, synthetic resins has been investigated [Krause et al "Mechanical properties of BIS-GMA resin short glass fiber composites", J. Biomed Materials Res. 23, 1195-1121 (1989)]. The authors of this paper were not able to demonstrate that improved strengths could be achieved in resins reinforced with fibres alone.

EP-A-0345581 discloses polymerizable compositions for the preparation of biocompatible compositions for medical and dental use, comprising 10 to 60 wt.% of monomers with radical-polymerizable double bonds, 8 to 40 wt.% of fibrous inorganic fillers with a fibre length of 10nm to 300µm and an aspect ratio of 2:1 to 300:1, 15 to 60 wt.% of inorganic particulate fillers having a particle size of 10nm to 200µm and up to 10 wt.% of adjuvant.

The present invention consists in a dental restorative composition in the form of a filled reinforced resin matrix, comprising 24 to 29 volume % of a dental synthetic resin; from 5 to 50% vol of fibres of 50µm or less diameter; from 10 to 65% vol of a first particulate filler having a particle size range of greater than 0.1 µm and up to 50µm; and from 5 to 25% vol of a second particulate filler having an ultimate particle size range of 10 to 100nm, said composition having a K_{lC} value of at least 1.2MN/m^{1.5}, volume % is based on the total volume of the adhesive.

Surprisingly it has been found that castable, synthetic resins with a particular combination of particulate and fibre reinforcement have improved strengths and fracture toughness over conventional castable, synthetic resins when used alone or only with fibres. The compositions of the invention, because of their improved mechanical properties have wider application than conventional castable synthetic resins.

The composition of the invention preferably comprises 25 to 35 vol %, of fibres of 50µm or less diameter, preferably 10 to 20µm diameter; from 40-50 vol % of a first particulate filler having an average particle size greater than 0.1 µm and up to 50µm and a preferred average size of less than 5µm; and from 10 to 20%, of a second particulate filler having an ultimate particle size range of 10 to 100nm, preferably approximately 40nm, and preferably a surface area of 50m²g⁻¹ or less.

By ultimate particle size we mean the particle size which is present in the matrix and may include agglomerates of the second particulate filler.

Suitable resins include acrylates, epoxy group containing resins and urethane group containing resins. Examples of such resins are Bis GMA, EGDMA and TEGDMA. Further examples of such resins include oligo dimethacrylates, oligo glycol dimethacrylates, oligo bisphenol A dimethacrylates, urethane dimethacrylates and urethane methacrylates.

A variety of fibre types can be utilised such as borosilicate ("E" glass) fibres, silica fibres, ceramic fibres, metal fibres and polymeric fibres. Long fibres (glass or carbon/graphite) can be used for fixed prosthodonture or implants. Very short fibres and platelets are particularly suitable for tooth restorations. The fibres can be mixed with the resins in a vacuum mixer to eliminate trapped air. For dental composite resins the fibres can be incorporated with the other particulate fillers and mixed with the resins in a vacuum mixer to eliminate trapped air.

For dental composite resins, the fibres preferably have an aspect (length:diameter) ratio of 50:l. Fibre length would generally be 5µm to 2.5mm, preferably 5µm to lmm and more preferably 50 to 500µm. For a diameter of 5 to 50µm the length would be 0.25 to 2.5mm for dental composite resins, unless the resins were to be used to manufacture bridges, where longer fibres would be employed.

Fibres of all types can be added to existing dental composite resin formulae in varying concentrations, resulting in a considerable increase in K_{lC} even at low concentrations such as 5%.

Fibres add substantially to the toughness and fracture resistance of the dental composite resin, due to the synergistic combination of filler particles and the fibres. An optimum is reached at approximately 30 to 50% fibre for each combination, until at high fibre concentration, 60 to 70%, the beneficial effect rapidly decreases with the corresponding decrease in other filler content.

Suitable particulate fillers include silica, porous amorphous silica (diatomaceous earth), feldspar, mica and talc.

Fillers, such as porous amorphous silica (diatomaceous earth), interlock very positively with the resin matrix, acting essentially as very short fibres, and signficantly increase K_{lC} over fine particles (glass, silica etc) at similar concentrations. They produce the highest K_{lC} values for anterior dental composite resins where small particles are essential to allow the surface to be polished to reproduce the high gloss of natural teeth. Compared to the normal microfine anterior dental composite resins, adding fillers can increase K_{lC} values by more than two times. The second particulate filler may be, e.g. pyrogenic silica or fine titania, zirconia or alumina.

Generally the reinforced dental composite resin compositions of the invention exhibit increased strength and toughness of between 200% and 400% relative to dental composite resins when this increase is measured as K_{lC} in MN/m 3/2.

The compositions of the invention may be precast, polymerised in situ, or part polymerised in situ, removed and post-cured.

### BEST METHODS OF CARRYING OUT THE INVENTION

Preferred composites are made up of 24 to 29% vol resin, approximately 30% vol fibres, approximately 30% vol of the first particulate filler and approximately 10% vol of the second particulate filler.

The following examples illustrate preferred embodiments of the invention.

### EXAMPLE 1

A reinforced resin of the following composition was prepared.

| | |
|---|---|
| Resin (12% Bis/GMA 12% TEGDMA) | 24% |
| Silica (average 17µm) | 29% |
| Pyrogenic Silica | 9% |
| Borosil Fibre (diameter 15µm length 400-800µm ) | 38% |

The paste is produced in small quantities by gradually introducing the premixed filler to the resin in a suitable size pestle and mortar. The added filler has to be thoroughly mixed before adding further filler in small increments. As the filler content percentage rises large shear forces are required. Preferably the paste should be placed under vacuum to eliminate trapped air in the mix.

The resultant composite can be chemically cured using a two paste system but preferably photocured as a single paste. The resin is mixed with an appropriate photoinitiator, either UV or visible light sensitive, an amine accelerator and a suitable amount of inhibitor to suit the particular application.

The resin of Example 1 has a plain strain fracture toughness (K_{lC}) of 1.6 HN/m^{1.5}.

### EXAMPLE 2

A reinforced resin of the following composition was prepared.

| | |
|---|---|
| Resin (Bis/GMA/TEGDMA) | 28% |
| Silica (average 17µm) | 27% |
| Pyrogenic Silica | 9% |
| Borosil Fibre (diameter 15µm length 400-800µm) | 36% |

The resin is produced in accordance with the method described in example 1. Increasing the resin content achieves a more optimum resin wetting of the fibres and increases the K_{lC} to 2.5 MN/m^{1.5} in the resin of this example.

### EXAMPLE 3

A reinforced resin of the following composition was prepared.

| | |
|---|---|
| Resin (Bis/GMA/TEGDMA) | 29% |
| Silica (average 17µm) | 36% |
| Pyrogenic Silica | 12% |
| Borosil Fibres (diameter 15µm length 400-800µm) | 23% |

The resin is produced in accordance with the method described in example 1. This resin has a K_{lC} of 1.2MN/m^{1.5}.

### EXAMPLE 4

A reinforced resin of the following composition was prepared.

| | |
|---|---|
| Resin (Bis/GMA/TEGDMA) | 29% |
| Silica (average 17µm) | 24% |
| Pyrogenic Silica | 14% |
| Borosil Fibres (diameter 15µm length 400-800µm) | 33% |

The resin is produced in accordance with the method described in example 1. Replacing some of the silica in the composition of example 3 with fibre, increasing the fibre content from 23% to 33%, doubles the K_{lC} to 2.8 MN/m^{1.5}.

### INDUSTRIAL APPLICATION

The resin matrices of the invention can be used in place of amalgam in dental restorations. They can also be used as a core material in dental restorations as dental implants and as a replacement for hydroxyapatite bone repair materials. Other tooth coloured dental restorative materials such as glass ionomers and cermets (low K_{lC}s) would be greatly strengthened by fibres, platelets or combinations thereof.

Longer glass or carbon/graphite fibres are suitable for strengthening dental composite resins for use in dental implants and fixed prosthodonture (crowns and bridges) either precast or polymerised in situ.

Matrices according to the invention can be used to provide improved electrical insulator casting resins.

Because of the biocompatibility and mechanical properties of the materials of the invention, they also find use as bone prostheses.

## Claims

1. A dental restorative composition in the form of a filled reinforced resin matrix, comprising 24 to 29 volume % of a dental synthetic resin; from 5 to 50% vol of fibres of 50µm or less diameter; from 10 to 65% vol of a first particulate filler having a particle size range of 0.1 greater than to 50µm; and from 5 to 25% vol of a second particulate µm and up filler having an ultimate particle size range of 10 to 100nm, said composition having a plain strain fracture toughness K_{lC} value of at least 1.2MN/m^{1.5}, volume % is based on the total volume of the composition.

2. A composition as defined in claim 1, wherein the fibres are borosilicate (E glass) fibres, silica fibres, ceramic fibres, metal fibres, carbon fibres or polymeric fibres.

3. A composition as defined in claim 1 or claim 2, wherein the amount of fibres is 25 to 35% vol.

4. A composition as defined in any one of claims 1 to 3, wherein the fibre diameter is 10 to 20µm.

5. A composition as defined in any one of claims 1 to 4, wherein the fibre length is 5µm to 2.5mm.

6. A composition as defined in any one of claims 1 to 5, wherein the fibre length is 5µm to 1mm.

7. A composition as defined in any one of claims 1 to 6, wherein the fibre length is 50 to 500µm.

8. A composition as defined in any one of claims 1 to 7, wherein the fibres have an aspect ratio of 50:1.

9. A composition as defined in any one of claims 1 to 8, wherein the first particulate filler is silica, a porous amorphous silica (diatomaceous earth), feldspar, Mica or talc.

10. A composition as defined in any one of claims 1 to 9, wherein the amount of first particulate filler is 40 to 50% vol.

11. A composition as defined in any one of claims 1 to 10, wherein the particle size range of the first particulate filler is less than 5µm.

12. A composition as defined in any one of claims 1 to 11, wherein the second particulate filler is pyrogenic silica or fine titania, zirconia or alumina.

13. A composition as defined in any one of claims 1 to 12, wherein the amount of second particulate filler is 10 to 20% vol.

14. A composition as defined in any one of claims 1 to 13, wherein the particle size of second particulate filler is approximately 40nm.

15. A composition as defined in any one of claims 1 to 14, wherein the surface area of the second particulate filler is 50m²g⁻¹ or less.

16. A composition as defined in any one of claims 1 to 15, wherein the resin is an acrylate, an epoxy-containing resin or a urethane-containing resin.

17. A composition as defined in claim 16, wherein the resin is an oligo dimethacrylate, an oligo glycol dimethacrylate, an oligo bisphenol A dimethacrylate, a urethane dimethacrylate or a urethane methacrylate.

18. A composition as defined in claim 16, wherein the resin is Bis GMA, EGDMA or TEGDMA.

19. A method for producing a dental restorative composition in the form of a filled reinforced resin matrix comprising 24 to 29 volume % of a dental synthetic resin, 5 to 50 vol.% of fibres of 50 µm or less in diameter, 10 to 65 vol.% of a first particulate filler having a particle size range of greater than 0.1 µm and up to 50 µm and from 5 to 25 vol.% of a second particulate filler having an ultimate particle size range of 10 to 100 nm, which comprises premixing the fibres and the particulate fillers, gradually adding the premixed fillers and fibres to the resin and mixing under vacuum to eliminate trapped air, to give a composition having a plain strain fracture toughness K_{lC} value of at least 1.2 MN/m^{1.5}.

## Patentansprüche

1. Zusammensetzung für die dentale Restauration in der Form einer gefülllten, verstärkten Harzmatrix, die 24 bis 29 Volumenprozent eines synthetischen dentalen Harzes, 5 bis 50 Volumenprozent von Fibern mit einem Durchmesser von 50µm oder kleiner; 10 bis 65 Volumenprozent eines ersten, partikulierten Füllers, der eine Partikelgröße von mehr als 0,1µm und bis zu 50µm aufweist; und 5 bis 25 Volumenprozent eines zweiten partikulierten Füllers, der eine ultimate Partikelgröße von mehr als 10nm bis 100nm aufweist, enthält, wobei diese Zusammensetzung einen Wert von K_{1C} der ebenen Bruchzähigkeit von wenigstens 1,2MN/m^{1.5} aufweist und sich die Volumenprozent auf das Gesamtvolumen der Zusammensetzung beziehen.

2. Zusammensetzung nach Anspruch 1, wobei die Fiber Borosilikat-Fibern (E-Glas), Silikat-Fibern, keramische Fibern, metallische Fibern Karbonfibern oder polymere Fibern sind.

3. Zusammensetzung nach Anspruch 1 oder 2, wobei die Menge der Fibern zwischen 25 und 35 Volumenprozent liegt.

4. Zusammensetzung nach einem der Ansprüche 1 bis 3, wobei der Durchmesser der Fibern zwischen 10 und 20µm liegt.

5. Zusammensetzung nach einem der Ansprüche 1 bis 4, wobei die Länge der Fibern zwischen 5µm und 2,5mm liegt.

6. Zusammensetzung nach einem der Ansprüche 1 bis 5, wobei die Länge der Fibern zwischen 5µm und 1mm liegt.

7. Zusammensetzung nach einem der Ansprüche 1 bis 6, wobei die Länge der Fibern zwischen 50 und 500µm liegt.

8. Zusammensetzung nach einem der Ansprüche 1 bis 7, wobei die Fibern ein Aspektverhältnis von 50:1 haben.

9. Zusammensetzung nach einem der Ansprüche 1 bis 8, wobei der erste partikulierte Füller Silikat, ein poröses. amorphes Silikat (diatomaceische Erde), Feldspat, Mica oder Talk ist.

10. Zusammensetzung nach einem der Ansprüche 1 bis 10, wobei die Menge des ersten partikulierten Füllers zwischen 40 und 50 Volumenprozent liegt.

11. Zusammensetzung nach einem der Ansprüche 1 bis 10, wobei die Partikelgröße des ersten partikulierten Füllers kleiner ist als 5µm.

12. Zusammensetzung nach einem der Ansprüche 1 bis 11, wobei der zweite partikulierte Füller pyrogenes Silikat oder feines Titanat, Zirkonat oder Aluminat ist.

13. Zusammensetzung nach einem der Ansprüche 1 bis 12, wobei die Menge des zweiten partikulierten Füllers zwischen 10 und 20 Volumenprozent liegt.

14. Zusammensetzung nach einem der Ansprüche 1 bis 13, wobei die Partikelgröße des zweiten partikulierten Füllers etwa 40nm beträgt.

15. Zusammensetzung nach einem der Ansprüche 1 bis 14, wobei die Oberflächengröße des zweiten partikulierten Füllers 50 m²/g oder weniger beträgt.

16. Zusammensetzung nach einem der Ansprüche 1 bis 15, wobei das Harz ein Acrylat, ein Epoxid enthaltendes Harz oder ein Urethan enthaltendes Harz ist.

17. Zusammensetzung nach Anspruch 16, wobei das Harz ein oligo-Dimethylacrylat, ein oligo-Glycol-Dimethylacrylat, ein oligo-Bisphenol A-Dimethylacrylat, ein Urethan-Dimethylacrylat oder ein Urethan-Methacrylat ist.

18. Zusammensetzung nach Anspruch 16, wobei das Harz Bis GMA, EGDMA oder TEGDMA ist.

19. Verfahren zur Herstellung einer Zusammensetzung für die dentale Restauration in der Form einer gefülllten, verstärkten Harzmatrix, die 24 bis 29 Volumenprozent eines synthetischen dentalen Harzes, 5 bis 50 Volumenprozent von Fibern mit einem Durchmesser von 50µm oder kleiner; 10 bis 65 Volumenprozent eines ersten, partikulierten Füllers, der eine Partikelgröße von mehr als 0,1µm und bis zu 50µm aufweist; und 5 bis 25 Volumenprozent eines zweiten partikulierten Füllers, der eine ultimate Partikelgröße von mehr als 10nm bis 100nm aufweist, enthält, das umfaßt ein Vormischen der Fibern und der partikulierten Füller, das allmähliche Zugeben der vorgemischten Fibern und Füller zu dem Harz und das Mischen unter Vakuum, um eingeschlossene Luft zu entfernen, um eine Zusammensetzung zu erzeugen, die einen Wert von K_{1C} der ebenen Bruchzähigkeit von wenigstens 1,2MN/m^{1,5} aufweist.

## Revendications

1. Une composition dentaire de reconstitution sous la forme d'une matrice de résine renforcée et chargée, comprenant de 24 à 29 % en volume d'une résine dentaire synthétique ; de 5 à 50 % en volume de fibres dont le diamètre est au plus égal à 50 µm ; de 10 à 65 % en volume d'une première matière de charge particulaire dont la grosseur de grain va de plus de 0,1 µm jusqu'à 50 µm ; et de 5 à 25 % en volume d'une seconde matière de charge particulaire dont la grosseur de grain finale va de 10 à 100 nm ; ladite composition ayant une valeur de ténacité à la fracture par déformation plane K_{1C} d'au moins 1,2 MN/m^{1,5}, et le pourcentage en volume étant basé sur le volume total du composite.

2. Une composition selon la revendication 1, dans laquelle les fibres sont des fibres de borosilicate (verre non alcalin), des fibres de silice, des fibres de céramique, des fibres métalliques, des fibres de carbone ou des fibres polymères.

3. Une composition selon la revendication 1 ou 2, dans laquelle la quantité de fibres est comprise entre 20 et 25 % en volume.

4. Une composition selon l'une quelconque des revendications 1 à 3, dans laquelle le diamètre de fibre est compris entre 10 et 20 µm.

5. Une composition selon l'une quelconque des revendications 1 à 4, dans laquelle la longueur de fibre va de 5 µm à 2,5 mm.

6. Une composition selon l'une quelconque des revendications 1 à 5, dans laquelle la longueur de fibre va de 5 µm à 1 mm.

7. Une composition selon l'une quelconque des revendications 1 à 6, dans laquelle la longueur de fibre va de 50 à 500 µm.

8. Une composition selon l'une quelconque des revendications 1 à 7, dans laquelle les fibres ont un rapport d'allongement de 50/1.

9. Une composition selon l'une quelconque des revendications 1 à 8, dans laquelle la première matière de charge particulaire est de la silice, une silice poreuse amorphe (diatomite), du feldspath, du mica ou du talc.

10. Une composition selon l'une quelconque des revendications 1 à 9, dans laquelle la quantité de première matière de charge particulaire est comprise entre 40 et 50 % en volume.

11. Une composition selon l'une quelconque des revendications 1 à 10, dans laquelle la grosseur de grain de la première matière de charge particulaire est inférieure à 5 µm.

12. Une composition selon l'une quelconque des revendications 1 à 11, dans laquelle la seconde matière de charge particulaire est de la silice pyrogénée, ou une poudre de dioxyde de titane, de zircone ou d'alumine.

13. Une composition selon l'une quelconque des revendications 1 à 12, dans laquelle la quantité de seconde matière de remplissage particulaire est comprise entre 10 et 20 % en volume.

14. Une composition selon l'une quelconque des revendications 1 à 13, dans laquelle la grosseur de grain de la seconde matière de remplissage particulaire est d'environ 40 nm.

15. Une composition selon l'une quelconque des revendications 1 à 14, dans laquelle la surface spécifique de la seconde matière de remplissage particulaire est de 50 m²g⁻¹ ou moins.

16. Une composition selon l'une quelconque des revendications 1 à 15, dans laquelle la résine est un acrylate, une résine époxy ou une résine uréthanique.

17. Une composition selon la revendication 16, dans laquelle la résine est un oligo-diméthacrylate, un oligo-glycol-diméthacrylate, un oligo-bisphénol A-diméthacrylate, un diméthacrylate uréthane, ou un méthacrylate uréthane.

18. Une composition selon la revendication 16, dans laquelle la résine est du Bis GMA, de l'EGDMA ou du TEGDMA.

19. Un procédé pour produire une composition dentaire de reconstitution sous la forme d'une matrice de résine renforcée et chargée comprenant de 24 à 29 % en volume d'une résine dentaire synthétique, de 5 à 50 % en volume de fibres de diamètre allant de 5 à 50 µm ou moins, de 10 à 65 % en volume d'une première matière de charge particulaire dont la grosseur de grain va de plus de 0,1µm jusqu'à 50 µm, et de 5 à 25 % en volume d'une seconde matière de charge particulaire dont la grosseur de grain finale va de 10 à 100 nm, qui comprend les étapes de faire un premier mélange avec les fibres et les matières de charge particulaires, ajouter progressivement les matières de charge et fibres du premier mélange à la résine et mélanger sous vide pour éliminer l'air emprisonné, afin d'obtenir une composition ayant une valeur de ténacité à la fracture par déformation plane K_{lC} d'au moins 1,2 MN/m^{1,5}.
